Europäisches Patentamt

European Patent Office

Office européen des brevets

(19)

(11) Publication number: **0 237 818**

**A1**

(12) # EUROPEAN PATENT APPLICATION

(21) Application number: **87102304.0**

(22) Date of filing: **18.02.87**

(51) Int. Cl.³: **A 61 F 2/10**

(30) Priority: **13.03.86 IT 1973786**

(43) Date of publication of application:
**23.09.87 Bulletin 87/39**

(84) Designated Contracting States:
**AT BE CH DE ES FR GB GR IT LI LU NL SE**

(71) Applicant: **Mazza, Marcello**
**Tigullio Rok's 128**
**Chiavari(IT)**

(71) Applicant: **Oddenino, Ruben**
**via Viviani 2**
**Milano(IT)**

(72) Inventor: **Mazza, Marcello**
**Tigullio Rok's 128**
**Chiavari(IT)**

(72) Inventor: **Oddenino, Ruben**
**via Viviani 2**
**Milano(IT)**

(74) Representative: **Gervasi, Gemma, Dr. et al,**
**NOTARBARTOLO & GERVASI Srl 33, Viale Bianca Maria**
**I-21100 Milano(IT)**

(54) **An artificial hair and a device for its implantation into the scalp.**

(57) An artificial hair having at its implanted end a conformation such as to ensure high retention of the implant and comprising a fixed knot (1) formed in the doubled fibre, a loop (2) and a short branch (3) intended for embedding during implantation.

Said hair is implanted into the scalp by a device comprising a needle which has a single point and a support saddle which engages the lower end of the hair for the implantation operation.

FIG. 1

EP 0 237 818 A1

- 1 -

AN ARTIFICIAL HAIR AND A DEVICE FOR ITS IMPLANTATION INTO THE
SCALP

This invention relates to an artificial hair and a device for its implantation into the scalp.

More particularly, the invention relates to an artificial hair having at that end to be implanted a conformation such as to ensure high retention of the implant, the retention of the implant being further assisted by the particular profile of the needle point of the device used for implantation.

Generally, the reaction of the human organism towards an artificial hair implanted into the scalp initiates with acute inflammation which appears as sero-fibrinous exudation and a leukocyte infiltration, followed by a foreign body reaction in the form of arrival of the lympho-monocyte phase, appearance of macrophages and giant cells, and subsequent connective-vascular reaction.

This reaction is related to the non-reabsorption of the foreign body introduced into the human organism, and this biological state determines the encystment of the foreign body within a fibrous capsule, so ensuring retention of the implant.

The extent of this retention is determined by the hair conformation at the implanted end and by the type of needle used for implantation.

Said retention is particularly ensured when the hair and needle according to the present invention are used for the implantation.

A first problem which arises in forming an artificial hair is the choice of type of fibre to be used.

In this respect, a foreign body for plastic surgery implantation must satisfy precise requirements to the highest degree, and in particular must be chemically and physically inert, non-inflammatory, non-allergenic, non-cancerogenous, non-toxic, non-biodegradable, and stable from the chemical, physical and dimensional aspects. The material must also be easily obtainable, easily sterilised and easily implantable. In practice, plastic surgery currently uses synthetic polymeric materials either of the thermoplastic or of the thermosetting type, and the choice of artificial hair is suitably made from polyamide, polypropylene and polyester fibres.

For the hairs according to the present invention, polyester fibres are preferably used of diameter between 0.05 and 0.1 mm.

Artificial hairs formed from synthetic polymeric fibres and devices for implanting them are known in the art.

In one known embodiment, that end of the hair to be implanted has an alpha-shaped knot conformation formed by microwave welding. This welding produces an irregular surface on the hair with carbon deposits which facilitate scalp infection only a short time subsequent to surgical implantation.

In addition, the weakness of the weld combined with the large diameter of the needle which carries the hair results in an implanted hair loss of 25-30% per year.

In another embodiment, that end of the hair to be implanted is in the shape of a ring made with a slip knot which, having a larger diameter than the hair fibre, means that a lacero-contusive wound is made in the scalp which is of such a diameter as to favour bacterial infiltration with consequent abnormal inflammatory reactions which do not allow uniform re-epithelization of the cutis, to result in expulsion of the hair after a certain time period.

In a recent embodiment (Italian patent application No. 24265 A/84), the artificial hair carries at its implanted end a self-tightening knot, for the implantation of which a needle is provided having its end in the form of two dovetail-shaped points.

This system has the following practical drawbacks:
-   lacero-contusive wounds due to the particular shape of the needle;
-   difficulty of implantation due to the need to fit the double fibre into the needle dovetail at the knot;
-   the ease of loosening the knot during implantation.

The drawbacks encountered when operating according to the known art are obviated by the artificial hair and implantation device according to the present invention, said artificial hair being characterised by comprising at its implanted end a fixed preformed knot, upstream of which there is provided a loop and downstream of which there extend two branches, one of which is cut to the correct size for incorporation during implantation and the other of which is cut to the correct hair size, said device being characterised by comprising an implantation needle provided with a single point and a support saddle.

These and further characteristics and advantages of the artificial hair and implantation device according to the present invention will be more apparent from the detailed description given hereinafter with reference to the figures which relate to preferred embodiments of the invention given by way of nonlimiting example.

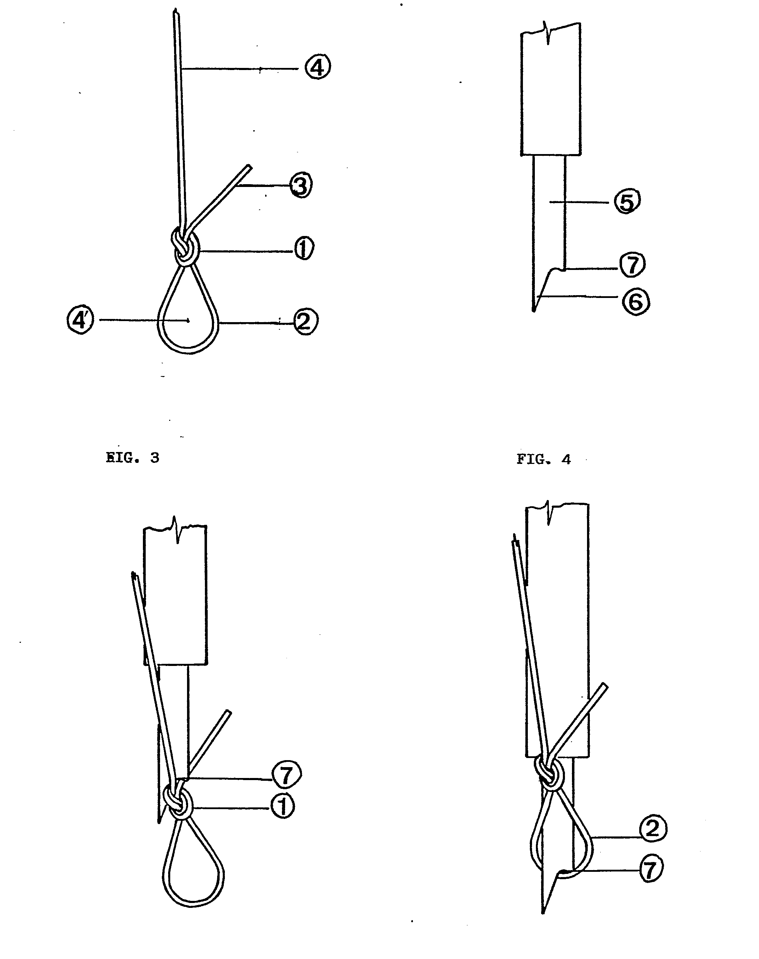

Figure 1 shows the conformation of the hair according to the invention. From it it can be seen that the double-fibre knot, of fixed type, is obtained by folding a fibre so that it becomes doubled, and knotting the double fibre in proximity to its folded end to give rise to the formation of the loop 2; the two branches 3 and 4 diverge by an angle of between 40 and 50° and preferably by an angle of 45°; the branch 3 is of such a length that on implantation it remains completely embedded in the scalp.

The three basic geometrical characteristics of the hair according to the invention, ie the loop 2, the knot 1 and the short branch 3, ensure an improved implant retention compared with hairs of the known art. In this respect:

- the loop 2 encloses the space 4', which becomes occupied during implantation by cell tissue which restrains the loop so retaining it on implantation;

- the knot 1 by virtue of its thickness constitutes a fixing element in that the cutaneous fibres close over it to restrain the branch 4 without leaving any discontinuity;

- the short branch 3, diverging from the branch 4, provides a further anchorage for the hair.

By virtue of these three elements, the loss of implanted hairs is very small, and practically zero.

Further advantages of the hair according to the invention compared with hairs of the known art are:

- the possibility of using a thinner needle as the needle need only engage the single fibre rather than the double fibre;

- the possibility of using a needle with a single point and a support saddle instead of a double-pointed dovetail needle, with the advantages which will be apparent hereinafter;

- the impossibility of loosening the knot and thus a greater retention of the implanted hair, a time saving in the surgical operation, and a lesser fibre wastage during implantation.

Figure 2 shows the particular profile of the tip of the needle

0237818

5 of the device according to the invention, which has a single point 6 and a support saddle 7.

Said point 6 extends over approximately one half of the needle cross-section, the support saddle 7 being provided in the other half, at the base of the point.

Said needle is constructed of music wire of diameter between 0.1 and 0.3 mm.

In the implantation operation, this type of needle produces a single hole which is formed gradually and much less traumatically than for example in the case of the double dovetail-pointed needle.

Furthermore, because of its geometrical characteristics, its elasticity and its thinness, it enables the implantation to be carried out quickly and without material wastage.

Finally, after implantation, the needle is automatically withdrawn by the device on which it is mounted, and leaves the scalp along the same path effected during penetration but in the reverse direction, so preventing further possible cutis trauma.

Figure 3 shows one method of engaging the hair by the needle for implantation. In this method, the saddle 7 is engaged by the knot 1. Figure 4 shows a further method of engaging the hair by the needle for implantation. In this further method, the saddle 7 engages the loop.

Figures 5, 6 and 7 show the device on which the needle 5 is mounted.

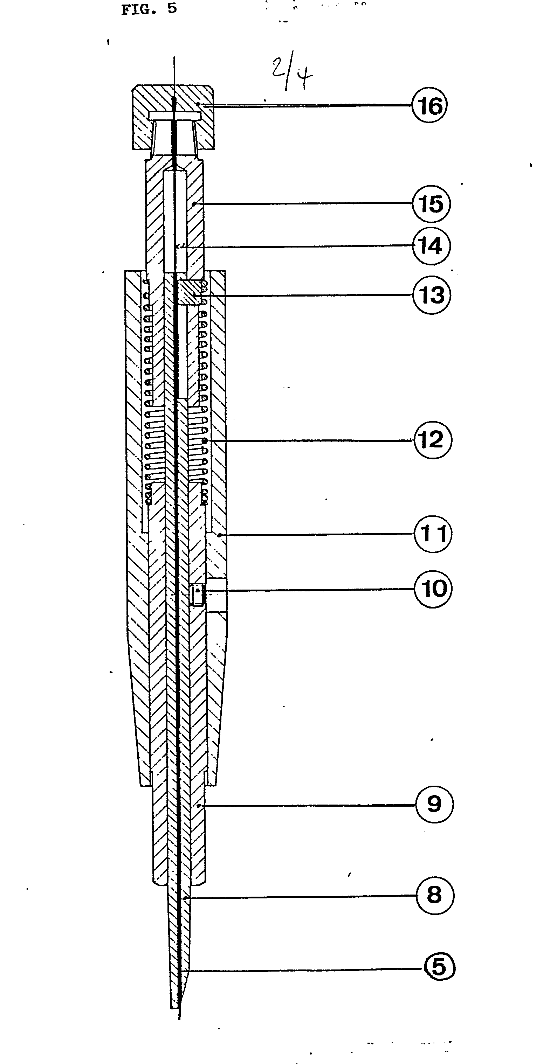

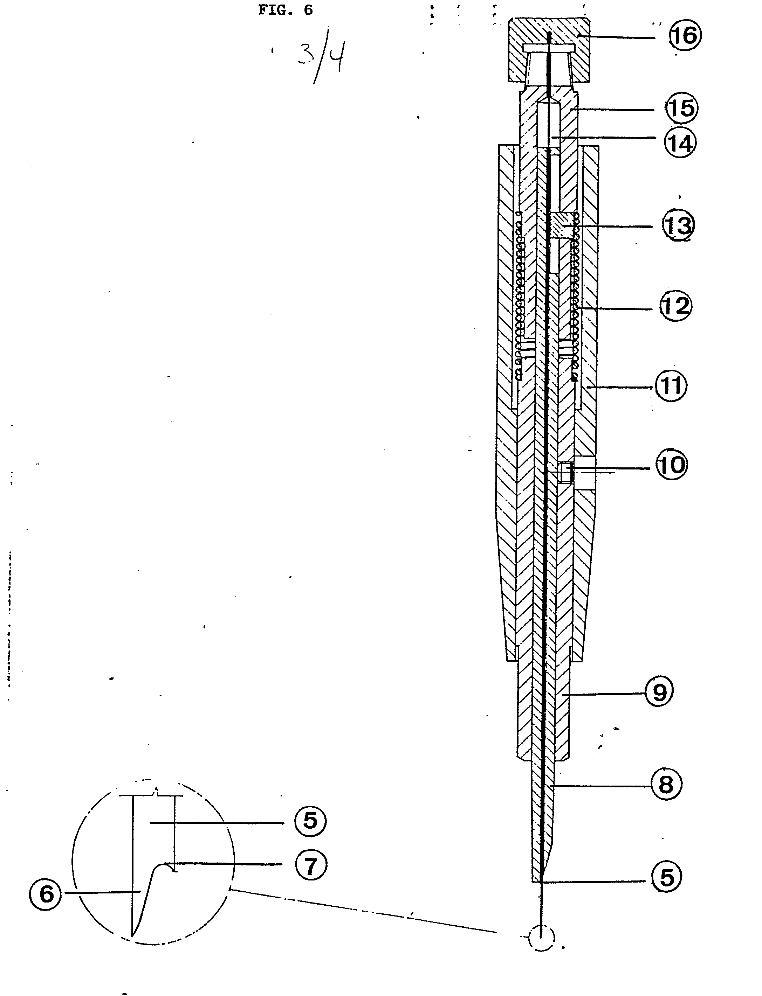

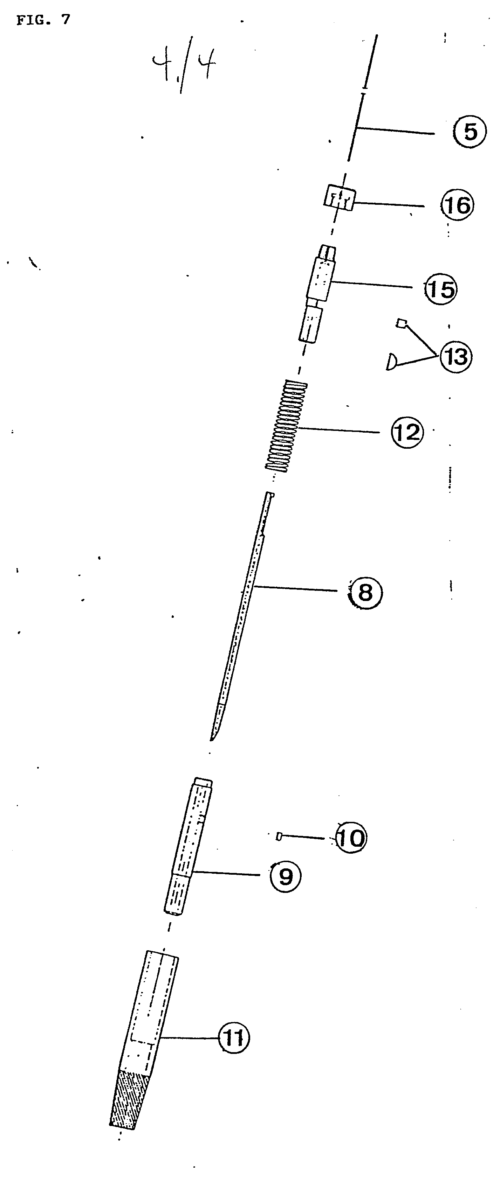

In Figures 5 and 6, the needle 5 is shown in its retracted position and extracted position respectively. Figure 7 is an isometric exploded view of the individual components of the device.

As can be clearly seen from said figures, the artificial hair implantation device according to the invention consists of the following elements: needle 5, needle carrier 8, holder 9 with retainer and guide for the loading spring 12, fixing screw 10, spring guide cap 11 for loading the spring, locking key 13, needle container 14, drive shaft 15 with shaft cover 16, with locking effect.

In order to make the implantation, the hair according to the invention is engaged by the support saddle 7 of the needle 5 at the knot 1 or at the loop 2, and is brought into contact with the cutis in a suitable disinfected and anesthetised region of the scalp.

The needle 5 is made to penetrate into the scalp by the operator pressing the shaft presser 16 of the device on which the needle is mounted. Said device is able to preprogramme the maximum travel of the needle 5, to carry the lower end of the hair into the scalp as far as the required point (approximately as far as the galea aponeurotica) as can be seen by comparing the situation of Figure 5 (needle 5 in retracted position) with the situation of Figure 6 (needle 5 in extracted position).

The needle 5 is then withdrawn, the implantation thus being concluded with the advantage over the self-tightening knotted hair that in this latter case after withdrawing the needle the hair must be pulled in order to tighten the knot to stabilise the implant, with the possibility of lacerating effects.

After withdrawal and suitable sterilisation, the needle 5 can be reused for subsequent implantation.

PATENT CLAIMS

1.      An artificial hair, characterised by comprising at its implanted end a fixed preformed knot, upstream of which there is provided a loop and downstream of which there extend two branches, one of which is cut to the correct size for incorporation during implantation and the other of which is cut to the correct hair size.

2.      An artificial hair as claimed in claim 1, characterised in that said knot and said loop are obtained by folding a fibre into doubled form and knotting the double fibre in proximity to its folded end, which forms the loop.

3.      An artificial hair as claimed in claim 1, characterised in that said two branches which extend from said knot diverge by an angle of between 40 and 50° and preferably by an angle of 45°.

4.      An artificial hair as claimed in claim 1, characterised by consisting of a fibre of synthetic polymeric material.

5.      An artificial hair as claimed in claim 1, characterised by consisting of a fibre chosen from the group comprising polyamide, polypropylene and polyester fibres.

6.      An artificial hair as claimed in claim 1, characterised by consisting of a hair of diameter between 0.05 and 0.1 mm.

7.      A device for implanting into the scalp an artificial hair claimed in the preceding claims, and comprising an implantation needle, characterised by possessing a single point and a support saddle.

8.      A device as claimed in claim 7, characterised in that said point of said needle extends over approximately one half of the cross-section of the needle, said support saddle being provided in the other half at the base of the point.

9.      A device as claimed in claim 7, characterised in that said needle is constructed of music wire of diameter between 0.1 and 0.3 mm.

10.      A device as claimed in claim 7, characterised in that the maximum travel of said needle is preprogrammed.

11.      A method for implanting artificial hairs claimed in claims 1 to 6 by the device claimed in claims 7 to 10, characterised in that said needle produces a single hole in a gradual manner during the implantation operation and then emerges by returning in the reverse direction along the same path as that taken during penetration.

FIG. 1

1/4

FIG. 2

FIG. 3

FIG. 4

FIG. 5

0237818

2/4

FIG. 6

3/4

0237818

FIG. 7

4/4

0237818

European Patent Office

| Category | Citation of document with indication, where appropriate, of relevant passages | Relevant to claim | CLASSIFICATION OF THE APPLICATION (Int. Cl.4) |
|---|---|---|---|
| X | DE-A-3 024 075 (YAMADA) <br> * entire document * | 1 | A 61 F 2/10 |
| A | | 2,3,7-11 | |
| X | GB-A-2 006 018 (YUGEN-GAISHA KONDO GIKEN) <br> * claims 1, 5; figure 1 * | 1 | |
| A | | 2-6 | |
| X | US-A-4 126 124 (MILLER) <br> * claim 1; figure 23 * | 1 | |
| A | | 7 | TECHNICAL FIELDS SEARCHED (Int. Cl.4) <br><br> A 61 F 2/00 |

### DOCUMENTS CONSIDERED TO BE RELEVANT

The present search report has been drawn up for all claims

| Place of search | Date of completion of the search | Examiner |
|---|---|---|
| BERLIN | 22-05-1987 | KANAL P K |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82